# DEMANDE DE BREVET EUROPEEN

(11) **EP 1 932 509 A1**
(43) Date de publication de la demande: **18.06.2008**
(21) Numéro de dépôt: 06291928.7
(22) Date de dépôt: 14.12.2006
(51) Int. Cl.: A61K 8/36, A61K 8/97, A61Q 5/00, A61Q 5/12, A61Q 7/00

(54) **Utilisation orale d'au moins un acide gras monoinsaturé pour améliorer la chevelure**

(71) Demandeur: L'ORÉAL, 75008 Paris (FR); Nestec S.A., 1800 Vevey (CH)
(72) Inventeur: Gueniche, Audrey, 92500 Rueil Malmaison (FR); Castiel, Isabelle, 06200 Nice (FR)
(74) Mandataire: Bernstein, Claire Jacqueline

(57) **Abrégé**

La présente invention se rapporte à l'utilisation orale d'au moins un acide gras mono-insaturé, ses sels et/ou esters, de préférence l'acide pétrosélinique, pour améliorer la qualité des cheveux et l'aspect de la chevelure.

## Description

La présente invention se rapporte à l'utilisation par administration orale d'au moins un acide gras monoinsaturé dans une composition renfermant un milieu cosmétiquement ou pharmaceutiquement acceptable, en tant qu'agent protecteur (traitement ou prévention) du cuir chevelu et des cheveux.

Le follicule pileux est formé de compartiments parfaitement individualisés, les uns d'origine dermique (gaine conjonctive et papille dermique), les autres de nature épithéliale (gaine épithéliale externe, gaine interne, tige pilaire et glande sébacée). La gaine conjonctive, synthétisée par des fibroblastes, est surtout une matrice extracellulaire formée de collagènes de type I et III, ainsi que de protéoglycannes. Traversée, dans le tiers inférieur, par un réseau de capillaires sanguins, elle se prolonge à la base du follicule, par la papille dermique, véritable agrégat de matrice extracellulaire. La papille dermique est constituée par des fibroblastes particuliers responsables de la synthèse locale de prostaglandine E2. Une membrane basale, composée de collagène de type IV, de laminine de type 1 et 5, de fibronectine et d'héparane sulfate protéoglycanes, sépare le compartiment dermique du compartiment épithélial. Le profil d'expression de la laminine 5 est toutefois interrompu à la périphérie du bulbe, soulignant ainsi une régionalisation de la jonction dermo-épidermique. Le compartiment épithélial peut, quant à lui, être séparé en quatre domaines distincts. A la base du follicule et entourant la papille dermique où se trouve la matrice, siège d'une intense activité mitotique. Cette matrice produit, avec des programmes de différenciation spécifiques, les trois grands domaines concentriques que sont la gaine externe, la gaine interne et la tige pilaire. Dans la partie supérieure du follicule se trouve la glande sébacée, formée de kératinocytes particuliers, les sébocytes responsables de la synthèse de sébum. La partie supérieure de la gaine externe est également caractérisée par la présence de cellules de Langerhans, véritables sentinelles du système immunitaire, et de cellules de Merkel remplies de neuropeptides et dont le rôle est encore méconnu (Revue dans « La vie révélée du follicule de cheveu humain »; Bernard BA, Medecine Sciences Vol 22(2), 2006).

La Demanderesse a réalisé depuis plusieurs années de nombreux tests cliniques et elle a pu ainsi déterminer les symptômes liés aux cheveux âgés. Ces symptômes sont en particulier un changement d'aspect de la fibre (cheveu fin, terne, sans tenu (mou), sans éclat, grisonnant), des cheveux qui ont tendance à chuter, qui se renouvellent moins vite. Ces manifestations résultent d'une désorganisation du réseau de collagène dans le derme du scalp, de l'apparition de produits de glycation (1) qui rigidifie le derme autour du follicule pileux entraînant des effets négatifs pour les follicules résidents et l'implantation de nouveaux cheveux, et de la réduction de la synthèse de sébum augmentant d'autant l'aspect terne et sans éclat du cheveu et favorisant la chute des cheveux (2, 3).
(1) Monnier VM, Cerami A. ACS Symposium Series 215 (1983) : 431-449
(2) Jaworsky C, Kligman AM, Murphy GF. Br J Dermatol 127 (1992) : 239-246
(3) Sueki H, Stoudemayer T et al. Acta Dermatol Venereol 79 (1999) : 347-350

Avoir des cheveux sains, vigoureux et nombreux tout au long de la vie est une ambition de la plupart des hommes et des femmes. De nombreux produits pour le traitement des cheveux sont disponibles. Cependant, aucune composition pour la voie orale ne permet à ce jour de lutter contre les altérations du cuir chevelu et/ou des cheveux liées apparaissant avec l'age et ne permet de protéger le « capital cheveu ».

La Demanderesse a mis en évidence que les acides gras monoinsaturés pouvaient permettre de lutter contre les altérations des cheveux vieillis.
Le vieillissement chronologique est un phénomène naturel qui se manifeste progressivement et plus ou moins tôt selon les individus. Les manifestations du vieillissement sur la chevelure
peuvent toutefois être accélérés par des facteurs endogènes, stress, maladies..., ou exogènes tels que la pollution, l'exposition aux UV.

Les acides gras mono-insaturés ont été décrits pour différentes applications telles que l'hydratation de la,peau sèche (EP 0.709 084 de L'Oreal).

EP 0 116 439 (Suntory) divulgue que certains acides gras ont une forte activité d'inhibition de la 5α-réductase de la flore bactérienne du cuir chevelu, et peuvent être utilisés de ce fait dans les produits toniques pour le traitement des pellicules et des démangeaisons du cuir chevelu. L'action de ces acides gras vise donc la flore bactérienne du cuir chevelu.

US 4,097,604 (Oxford Hill) indique qu'un sel de différents acides gras est actif contre les micro-organismes pathogènes de la cavité buccale, permettant ainsi de diminuer l'incidence de ces bactéries sur d'apparition de periodontoses. L'action de ces acides, gras est dans ce cas limitée à la flore buccale.

EP 0 355 842 (Sansho Seiyaku) décrit une crème de soin destinée à prévenir la pigmentation causée par une surproduction de mélanine, ladite crème pouvant renfermer de l'acide pétrosélinique.

EP 1 013 178 (Unilever) décrit l'utilisation d'acide pétrosélinique comme agent anti-inflammatoire et pour traiter les signes cutanés du vieillissement (ride, peau flasque, taches de sénescence).

Cependant aucun de ces documents ne laissait présager de l'activité des acides gras mono-insaturés sur la qualité de la fibre capillaire et, ainsi de la chevelure.

Ainsi, la présente invention se rapporte à l'utilisation orale d'au moins un acide gras mono-insaturé, ses sels ou ses esters, pour prévenir et/ou traiter les altérations du cuir chevelu (scalp).
Les altérations du cuir chevelu sont notamment les fibroses des tissus entourant les follicules pileux.

La Demanderesse a en effet pu mettre en évidence que les acides gras monoinsaturé conduisaient à :
- une restructucturation du réseau collagénique notamment visible par coloration au Trichorme Masson sur une coupe de peau d'un individu supplémenté avec de l'huile de coriandre, en effet, cette huile a comme activité la diminution de l'activité collagénase et élastase (mesuré par dosage enzymatique) ;
- une diminution des protéines glyquées retrouvées dans le derme et l'épiderme (diminution des dérivés carboxylmethyl-lysine) ;
- une diminution des marqueurs de stress et d'inflammation (tels que Hsp 70 et TNF-alpha dont la mesure peut être réalisée avec des anti-corps spécifiques) or le stress oxydatif du tissu environnant des follicules pileux conduit à une altération des cheveux, voire de leur chute.
Ils préviennent également l'altération du derme par les enzymes tissulaires telles que les collagènases, élastases et gelatinases et conduident ainsi à un profil cutané sain, sans fibrose, favorable à la pousse d'un cheveu de bonne qualité.

L'utilisation d'au moins un acide gras mono-insaturé, de ses sels ou esters permet également de limiter la chute des cheveux et/ou augmenter la densité de la chevelure.
La densité d'une chevelure se détermine en fonction du nombre de cheveu pour une surface de scalp donnée.

L'utilisation selon l'invention permet de prévenir et/ou limiter la formation de cheveux fins et/ou ternes et/ou cassants et/ou mous.

L'utilisation selon l'invention permet également d'améliorer la qualité des fibres kératiniques, notamment en favorisant la pousse de cheveux brillants et/ou épais et/ou vigoureux.

On entend par acide gras mono-insaturé au sens de la présente invention, un acide gras comprenant une unique double liaison.
Il s'agit plus particulièrement d'acides gras à longues chaînes. Les acides gras mono-insaturés convenant à l'invention sont les acides gras mono-insaturés comportant 12 à 22 atomes de carbone.

Les acides gras mono-insaturés peuvent être sous forme acide, ou de sel ou encore sous forme de dérivés notamment d'esters ou d'amides d'acides gras.

Lorsqu'ils se présentent sous forme de sel, les acides gras mono-insaturés peuvent plus particulièrement être des sels cosmétiquement acceptables, c'est-à-dire, des sels inorganiques tels que des sels d'ammonium ou de métaux alcalins (lithium, potassium, sodium) d'alcalino terrreux (magnesium, calcium) ou des sels d'aluminium. En particulier, les acides gras mono-insaturés se présentent sous forme de sel de calcium.

Les acides gras peuvent aussi se présenter sous forme d'ester, alors, ils peuvent être estérifiés avec le glycérol (mono-, di- ou tri-acyl), un alcool comme les alcools méthylique et éthylique, un sucre, un tocophérol, un tocotriénol, un stérol ou encore un alcool gras.
A titre d'acides gras mono-insaturés, on peut citer l'acide pétrosélinique (ou acide delta-6-cis-octadecenoique en C18), l'acide palmitoleique (ou acide cis-9-hexadécénoïque, en C16) ou l'acide oléique (acide cis-9-octadécénoïque, en C18).
Il est entendu que le choix des acides gras est effectué en tenant compte de la finalité de la composition les comportant, et notamment, son mode d'administration, orale ou par voie aérienne. Par voie aérienne, on entend les voies aériennes supérieures (comme les fosses nasales, les sinus, la bouche).

Parmi les acides gras mono-insaturés, on utilise particulièrement l'acide oléique et l'acide pétrosélinique. Convient tout particulièrement à l'invention l'acide pétrosélinique.

Selon une variante de l'invention, le ou les acides gras mono-insaturés sont utilisés sous une forme isolée, c'est-à-dire après extraction de leur source d'origine.

Selon une autre variante de l'invention, le ou les acides gras mono-insaturés proviennent d'extraits végétaux tels que des huiles.
Ainsi l'invention se rapporte notamment à l'utilisation d'une huile riche en acide gras mono-insaturé.

On peut notamment utiliser des huiles riches en acide pétrosélinique qui ont plus particulièrement choisies parmi les huiles d'ombellifère.
On entend par huile riche en acide pétrosélinique, une huile comprenant au moins 40% d'acide prétrosélinique.
Les ombellifères sont des plantes dont les fleurs sont disposées en ombelles, les espèces particulièrement riches en acide pétrosélénique sont les Umbellifarea-Apiacea et Araliaceae. Les plantes du genre Thapsia sont également des sources d'acide pétrosélénique (Avato et al, Lipids, 2001, 36, 845). Les espèces de préférence utilisées dans l'invention sont le coriandre, le cerfeuil, la carotte, le céleri, le cumin, le carvi, le persil et l'aneth. L'huile d'ombellifère utilisée selon l'invention peut être extraite de la graine de ces ombellifères, par exemple par broyage ou pressage, puis raffinage. L'huile d'ombellifère a une teneur en acide pétrosélinique qui varie selon la graine d'ombellifère d'où elle est extraite. Pour une même ombellifère, la teneur en acide pétrosélinique varie aussi selon le pays d'origine de l'ombellifère et selon l'extraction qui peut être plus ou moins complète.

L'acide pétrosélénique est également un composé abondant (environ 48%) de l'huile de graine de Gernium sanguneum (Tsevegsuren et al, Lipids, 2004, 39, 571).

La teneur en acide gras monoinsaturé ou ses dérivés dans les compositions pour la voie orale utilisables selon l'invention sera telle que les doses journalières sont comprises entre 0,5 et 2500 mg/j, notamment entre 5 et 500 mg/j.

Les compositions orales utilisables selon l'invention présentent préférentiellement un support ingérable.
Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.
Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des produits alimentaires de type confiserie, chocolat, céréales, des aliments pour animaux en particulier domestiques, des comprimés, gélules ou tablettes, des suppléments oraux sous forme sèche.

Bien entendu, les compositions orales selon l'invention peuvent en outre contenir plusieurs autres actifs.

Ces actifs pourront être choisis parmi ceux permettant de lutter contre le vieillissement du cuir chevelu et améliorer l'aspect de la chevelure, en particulier, on peut utiliser la taurine, la vitamine B6, la cystéine, la vitamine B1 (biotine), la vitamine B12 (bepanthène) et la gélatine. Les compositions orales pourront en outre comprendre un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des antocyanines.

Selon un autre de ses aspects, l'invention a pour objet un procédé cosmétique pour améliorer la qualité de la chevelure et de la fibre capillaire comprenant au moins une étape d'ingestion d'une composition orale comprenant, dans un support physiologiquement acceptable, au moins un acide gras mono-insaturé et/ou un ester d'acide gras mono-insaturé et/ou un de leurs sels.
De préférence, l'acide gras mono-insaturé est l'acide pétrosélinique.

Le procédé cosmétique de l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Selon encore un autre de ses aspects, l'invention a pour objet l'utilisation d'au moins un acide gras monoinsaturé, ses sels ou ses esters pour la fabrication d'une composition cosmétique ou dermatologique destinée à traiter ou prévenir les pathologies du cuir chevelu et/ou des cheveux.

### Exemple 1 - GEL UNIDOSE

| **Principe actif** | **% pds** |
|---|---|
| Huile de pépins de cassis | 10 |
| Huile de coriandre | 10 |
| Lycopene | 10 |
| *Lactobacillus johnsonii* | 10¹⁰ cfu |

| **Excipient** | |
|---|---|
| Sirop de sucre | 50 |
| Maltodextrine | 17 |
| Gomme Xanthane | 0,8 |
| Benzoate de sodium | 0,2 |
| Eau | QSP 100 |

On peut prendre une dose de 200 à 400 ml par jour.

### Exemple 2 - CAPSULE

| | **mg/capsule** |
|---|---|
| Huile de fenouil | 300 |
| Huile de pépins de raisin *Lactobacillus paracaseï* | 200 10¹⁰ cfu |
| Glycérine | 150 |
| stéarate de magnésium | 0,02 |
| arôme naturel | 0,01 |

On peut prendre une à trois de ces capsules par jour.

### Exemple 3

On adjoint à la formulation de l'exemple 2 un complexe vitaminique comportant 60 mg de vitamine C, 100 µg de vitamine E et 6 mg de β-carotène.

### Exemple 4

On adjoint à la formulation de l'exemple 2, un complexe vitaminique comportant 100 mg de vitamine C, 100 µg de vitamine E et 6 mg de lycopène par capsule.

### Exemple 5

On adjoint à la formulation de l'exemple 2, un complexe vitaminique comportant 100 mg de vitamine B1, B6, B12, 100 µg de taurine et 6 mg de gélatine par capsule.

### Exemple 6 - CAPSULE

| | **mg/capsule** |
|---|---|
| Vitamine C | 60 |
| Huile de carotte | 300 |
| Huile de coriandre | 200 |
| Glycérine | 150 |
| stéarate de magnésium | 0,02 |
| arôme naturel | qsp |

On peut prendre une à trois de ces capsules par jour.

### Exemple 7

On adjoint à la formulation de l'exemple 6 un complexe vitaminique comportant 60 mg de vitamine C, 100 µg de vitamine E et 6 mg de β-carotène.

### Exemple 8

On adjoint à la formulation de l'exemple 6 un complexe vitaminique comportant 100 mg de vitamine C, 100 µg de vitamine E et 6 mg de lycopène par capsule.

### Exemple 9

On adjoint à la formulation de l'exemple 6 un complexe vitaminique comportant 100 mg de vitamine B1, B6, B12, 100 µg de taurine et 6 mg de gélatine par capsule.

## Revendications

1. Utilisation par voie orale d'au moins un acide gras mono-insaturé, ses sels ou ses esters, pour prévenir et/ou traiter les altérations du cuir chevelu.

2. Utilisation selon la revendication 1, pour prévenir et/ou traiter les fibroses des tissus entourant les follicules pileux.

3. Utilisation selon la revendication 1 ou 2 pour limiter la chute des cheveux et/ou augmenter la densité de la chevelure.

4. Utilisation selon la revendication 1 ou 2 pour prévenir et/ou limiter la formation de cheveux fins et/ou ternes et/ou cassants et/ou mous.

5. Utilisation selon l'une quelconque des revendications précédente pour améliorer la qualité des fibres kératiniques.

6. Utilisation selon la revendication 5, pour favoriser la pousse de cheveux brillants et/ou épais et/ou vigoureux.

7. Utilisation selon l'une quelconque des revendications précédentes, **caractérisée en ce que** l'acide gras mono-insaturé est utilisé sous forme isolé ou dans un extrait végétal.

8. Utilisation selon l'une quelconque des revendications précédentes, **caractérisée en ce que** la teneur en acide gras mono-insaturé son sel et/ou son ester est comprise entre 0,0001 et 30 % en poids par rapport au poids total de la composition.

9. Utilisation selon l'une quelconque des revendications précédentes, **caractérisée en ce que** l'acide gras mono-insaturé est l'acide pétrosélinique.

10. Utilisation selon la revendication 9, **caractérisée en ce que** l'acide pétrosélinique est utilisé sous forme d'huile d'ombellifère ou de Gernium sanguneum.

11. Utilisation selon la revendication 10, **caractérisée en ce que** l'huile d'ombellifère est choisie parmi les huiles de graines de coriandre, cerfeuil, carotte, céleri, cumin, carvi, persil et aneth.

12. Utilisation selon l'une quelconque des revendications précédentes, **caractérisée en ce que** ledit acide monoinsaturé est utilisé en association avec au moins un actif choisi parmi les vitamines B1, B3, B5, B6, B8, B12, C, D, E, ou PP, la niacine, les caroténoïdes, les polyphénols et minéraux, les acides aminés, les catéchines, les oligo-proanthocyanidines, les acides aminés soufrés, les acides gras polyinsaturés oméga 3 et 6, la gélatine

13. Procédé cosmétique pour améliorer la qualité de la chevelure et de la fibre capillaire **caractérisé en ce qu'**il comprend l'ingestion d'une composition orale comprenant au moins un acide gras mono-insaturé, son sel et/ou son ester.

14. Procédé selon la revendication 13, **caractérisée en ce que** l'acide gras mono-insaturé est l'acide pétrosélinique.
